Europäisches Patentamt

European Patent Office

Office européen des brevets

(19)

(11) Publication number: **0 101 921**
**B1**

# EUROPEAN PATENT SPECIFICATION

(12)

(45) Date of publication of patent specification: **16.04.86**

(51) Int. Cl.⁴: **C 07 C 85/18**

(21) Application number: **83107291.3**

(22) Date of filing: **25.07.83**

(54) Amines via the amination of olefins using offretite.

(30) Priority: **30.07.82 US 403889**

(43) Date of publication of application:
**07.03.84 Bulletin 84/10**

(45) Publication of the grant of the patent:
**16.04.86 Bulletin 86/16**

(84) Designated Contracting States:
**AT BE CH DE FR GB IT LI LU NL SE**

(56) References cited:
**EP-A-0 026 071**
**EP-A-0 039 918**
**EP-A-0 077 016**

**J.A. RABO: "Zeolite chemistry and catalysis",**
**1976, edited by Jule A Rabo, American**
**Chemical Society, Washington, D.C., USA**

The file contains technical information
submitted after the application was filed and
not included in this specification

(73) Proprietor: **AIR PRODUCTS AND CHEMICALS,
INC.**
**Route no. 222**
**Trexlertown Pennsylvania 18087 (US)**

(72) Inventor: **Ambs, William Joseph**
**325 Dartmouth Avenue Apartment M6**
**Swarthmore, PA 19081 (US)**
Inventor: **Deeba, Michel**
**1080G Coldstream Circle**
**Emmaus, PA 18049 (US)**
Inventor: **White, James Ferguson**
**1315 Amstel Way**
**West Chester, PA 19380 (US)**

(74) Representative: **Sandmair, Kurt, Dr. et al**
**Patentanwälte Schwabe, Sandmair, Marx**
**Stuntzstrasse 16**
**D-8000 München 80 (DE)**

## Description

Technical field
This invention relates to a process for producing amines by the reaction of ammonia or ammonia type compound with an olefin.

Background of the prior art
The earliest work relating to the manufacture of amines by the amination of olefins particularly ethylamines by the amination of ethylene, appears to have been done by Teter et al as noted in U.S. Patents 2,623,061; 2,479,879; 2,417,892; 2,381,470; 2,658,041; 2,381,709; 2,392,107 and 2,398,899. These patents show that ammonia can be made to react with olefins, e.g. ethylene to produce amines. As noted by others through improvements in the process, substantial quantities of polyamines and polyolefins were produced by the Teter et al catalyst which is a metal supported on a spinel type support, silica and diatomaceous earth and the like.

Olin et al in U.S. Patents 2,422,631 and 2,422,632, discloses a process for producing amines and amides by the reaction of a monounsaturated olefin, carbon monoxide and an amine or ammonia. The catalyst used is a combination of a dehydrating and a hydrogenation catalyst, e.g. nickel and activated alumina, copper and silica gel, etc.

Whitman, U.S. 2,501,556 discloses a liquid phase reaction of ammonia and ethylene in the presence of an alkali metal catalyst to form ethylamine.

McClain, U.S. 3,412,158 discloses a gas phase process for producing primary alkyl amines from low molecular weight olefins and ammonia by carrying out the gas phase process in the presence of a noble metal containing catalyst at temperatures of from 90—175°C, at pressures of from atmospheric to 143 bar.

Peterson et al., U.S. 4,289,908 shows that aluminosilicates and particularly zeolites can be used for effecting amination of olefins at temperatures of 200—500°C, pressures from 21—430 bar. High yields and selectivity are reported.

The use of acidic alumino-silicates as catalysts in an amination process of olefines with ammonia is described in the EP—A—0 039 918 and the use of selected zeolites for the amination process is shown in the EP—A—0 077 016.

However, the catalysts of the prior art show some substantial disadvantages, especially in respect of acitivity, effectivity, selectivity, catalyst charge and lifetime.

Summary of the invention
This invention relates to an improved process for the preparation of amines by the vapor phase catalytic reaction of a $C_{2-4}$ olefin and ammonia wherein the amination is carried out in the vapor phase with a catalyst comprising an acidic alumino silicate under amination conditions which include a temperature in the range of about 300—400°C, a pressure in the range of about 43—78 bar, a mole ratio of ammonia to $C_{2-4}$ olefin of about 1—10:1 and a gas hourly space velocity of about 500—5000, characterized in that as acidic alumino silicate an acidic offretite which is rare earth or hydrogen ion exchanged is used.

There are several advantages either singly or combination associated with the invention as compared to many prior art processes. These advantages include:
an ability to produce $C_{2-4}$ alkyl amines, and particularly ethylamines in high selectivity, without producing substantial by-products. The production of polymers has been a serious problem with many of the prior art processes;
an ability to use a gas phase reaction which permits a high production rate in producing amines;
an ability to reduce the amount of by-product in the form of by-product nitrogen compounds e.g. nitriles as compared to prior art processes;
an ability to aminate multiple olefins, e.g. propylene ethylene without effecting a catalyst change in the reactor;
an ability to obtain good to high conversion of olefin to amine by virtue of the use of the particular catalyst under the conditions employed and to obtain good catalyst life; and
an ability to obtain a low aging rate with the particular offretite catalysts.

Detailed description of the invention
In the animation process, the olefin is reacted with ammonia to produce the amine. Higher molecular weight olefins, e.g. propylene and butylene are more difficult to aminate with these catalysts, but in contrast to similar zeolites, e.g., clinoptilolite and erionite, offretite is more active and will effect amination of the higher olefins.

In the process, ammonia is reacted with the $C_2$—$C_4$ olefin at temperature broadly from about 300—400°C Lower temperatures result in lower conversion, and higher temperatures often result in lower selectivity which generally appears in the form of a nitrile or unreacted hydrocarbon.

Pressures suited for practicing the invention are from about 21—143 bar with preferred pressures of from about 43—78 bar. Generally, pressures lower than about 43 bar result in poor conversion of ethylene to amine. On the other hand, as the pressure is increased above about 107 bar, conversion is increased only slightly. In order to minimize the recovery problems associated with separating the amine from the

2

reactants and other by-products, higher conversions can be waived and pressures of from about 43—78 bar used as this permits extremely high selectivities, e.g. greater than 95%.

Another important variable in the gas phase amination of the olefin is the mole ratio of ammonia to olefin in the feed. Generally, the mole ratio can be from about 0.2—20 moles ammonia per mole of olefin with preferred ranges being from about 1—10 to 1 ammonia to olefin. Mole ratios higher than about 10:1 of course require greater energy expenditure to handle the larger quantities of gas that pass through the reactor, and there seems to be no significant advantage in terms of increased selectivity or conversion at these higher ratios of ammonia to olefin. On the other hand, as the mole ratio of ammonia to olefin falls below about 0.2, there is a loss of selectivity to the amine.

The gas hourly space velocity (GHSV), which has units of volume of gas per volume of catalyst in unit time i.e. (cc gas at STP)/(cc catalyst hours$^{-1}$), can be from about 500—5,000 with a preferred range being from about 750—2,000. As the space velocity is increased toward the upper end of the range, conversion generally falls dramatically, particularly above about 3,000. On the other hand, as the space velocity approaches the lower level of 500, selectivity decreases and by-products form in greater concentration.

One of the important factors in achieving high selectivity to amines without the concomitant production of substantial amounts of by products in the form of polymer, e.g. polyethylene; or hydrocarbons or nitrogen compounds as encountered in the prior art processes including those using zeolites, is the use of a highly acidic offretite. This zeolite is highly selective for the amination of ethylene and propylene and produces unique results in terms of conversion. For example chabazite, which is a natural zeolite, gives lower conversion and selectivity. So does clinoptilolite and erionite and, from an energy viewpoint, higher selectivity and conversions can be achieved with the acidic offretite at lower temperatures. Thus, in the reaction, at least about 80% by weight and preferably all of the catalyst employed should be an acidic offretite. It is believed the high acidity and narrow pore size of this material plays an important role in the catalyst activity and selectivity.

Various ions can be incorporated into the offretite by conventional techniques to provide acidity. Trivalent ions, and particularly the rare earth metals, i.e. lanthanum, neodymium, praseodymium, are well suited for preparing exchanged zeolites. Other cations which may be used in place of the original metal cations of the offretite include hydrogen, barium, beryllium, calcium, cerium, magnesium, potassium, silver and zinc. However, sodium and potassium tend to reduce activity presumably due to the lowering of the acidity of the catalyst.

The alumino-silicate, offretite, of the present invention may be employed alone or in combination with various binder or matrix materials (organic or inorganic materials, resins, clays, gels or the like). The physical form of the catalyst may be adapted to use in any conventional catalytic system (e.g. slurry or fluidized bed systems, beads, pellets or the like for use in fixed or moving bed catalysts). As is known, the hydrogen form of offretite can be prepared from the ammonium form and for purposes herein is referred to as the hydrogen form. The preferred ions for the exchanged offretite are lanthanum and hydrogen as the exchanged offretite provide good yields, good selectivity and good catalyst life.

The following examples are provided to illustrate preferred embodiments of the invention and are not intended to restrict the scope thereof.

Example 1

A series of amination reactions were carried out using a reactor consisting of a stainless steel tubing 22,86 cm long of 1,90 cm outside diameter and 0,79 cm inside diameter. The stainless steel reactor was mounted inside a close fitting block of Inconel metal which was instrumented for temperature control. A thermowell extending axially through the reactor was used to measure reactor temperature.

The catalyst volume for the reactor was approximately 6 cubic centimeters, and the catalyst was held in place by a quartz wool plug. The catalysts were sieved to pass through a 12 U.S. standard sieve but retained on an 18 mesh sieve.

In the runs the experimental catalysts were prepared by conventional techniques, as for example, by the technique set forth in U.S. 3,758,539, which technique is incorporated by reference. Such H-form offretite catalysts used here were generally prepared by synthesizing the tetramethyl ammonium form followed, pelletizing and heat treating at 537°C. Comparisons were made against Linde SK-500 zeolite (REY—a rare earth exchanged zeolite), and H forms of clinoptilolite and erionite.

Table 1 represents results for the various catalysts tried including reactor conditions. Table 2 is an average summary of work carried out in the manner of Table 1 including runs 1—8.

TABLE 1

Ethylene conversion over H-offretite

| Run | Olefin | Catalyst | T, °C | bar | GHSV | Moles $NH_3$/ olefin | Conv. (%) | % Selectivity | | |
|---|---|---|---|---|---|---|---|---|---|---|
| | | | | | | | | Monoamine | Diamine | Other |
| 1 | Ethylene | H-offretite | 320 | 54 | 2000 | 4.2 | 4.0 | 100 | — | |
| 2 | " | " | 340 | " | 2000 | 4.1 | 7.2 | 95 | 4 | |
| 3 | " | " | 360 | " | 2000 | 4.0 | 12.3 | 93 | 5 | |
| 4 | " | " | 380 | " | 2000 | 4.2 | 16.9 | 90 | 6 | |
| 5 | " | " | 340 | " | 2000 | 4.2 | 7.3 | 95 | 4 | |
| 6 | " | " | 340 | " | 1180 | 1.8 | 10.1 | 88 | 11 | |
| 7 | " | " | 350 | " | 1180 | 1.7 | 11.9 | 86 | 12.5 | |
| 8 | " | " | 360 | " | 1180 | 1.7 | 14.6 | 84 | 14 | |
| 9 | Propylene | " | 300 | " | 2000 | 4.0 | 0.55 | 100 | — | |
| 10 | " | " | 320 | " | 2000 | 4.0 | 1.60 | 100 | — | |
| 11 | " | " | 340 | " | 2000 | 4.0 | 3.67 | 95.5 | 4.5 | |
| 12 | " | " | 350 | " | 2000 | 4.0 | 4.48 | 95.0 | 5.0 | |
| 13 | " | " | 350 | " | 1000 | 4.0 | 6.40 | 93 | 7 | |
| 14 | " | " | 350 | " | 500 | 4.0 | 7.20 | 90 | 8 | |
| 15 | " | " | 300 | " | 2000 | 2.0 | 1.0 | 95 | 5 | |
| 16 | " | " | 320 | " | 2000 | 2.0 | 2.1 | 96.4 | 3.6 | |
| 17 | " | " | 340 | " | 2000 | 2.0 | 3.6 | 95 | 5 | |
| 18 | " | " | 350 | " | 2000 | 2.0 | 4.2 | 94 | 6 | |
| 19 | " | " | 350 | " | 1000 | 2.0 | 5.2 | 90.2 | 6.5 | 3.7 |

TABLE 1 (Cont'd)

| Run | Olefin | Catalyst | T, °C | bar | GHSV | NH₃/ olefin | Conv. (%) | % Selectivity | | |
|-----|--------|----------|-------|-----|------|-------------|-----------|-----------|--------|-------|
| | | | | | | | | Monoamine | Diamine | Other |
| 20 | " | " | 350 | " | 500 | 2.0 | 5.2 | 87 | 7 | 6 |
| 21 | Ethylene | Clinoptilolite | 340 | " | 2000 | 4.5 | 4.4 | 100 | — | |
| 22 | " | " | 340 | " | 2000 | 4.9 | 6.6 | 100 | — | |
| 23 | " | " | 360 | " | 2000 | 4.6 | 11.4 | 100 | — | |
| 24 | " | " | 370 | " | 2000 | 4.4 | 13.7 | 93.1 | 6.9 | |
| 25 | " | " | 370 | " | 2000 | 4.6 | 14.9 | 91.4 | 8.6 | |
| 26 | " | " | 380 | " | 2000 | 4.4 | 14.8 | 91.9 | 8.1 | |
| 27 | " | " | 380 | " | 2000 | 4.7 | 14.4 | 92 | 8 | |
| 28 | " | " | 390 | " | 2000 | 4.4 | 15.7 | 91 | 9 | |
| 29 | " | " | 400 | " | 2000 | 4.7 | 18 | 90.2 | 9.8 | |
| 30 | " | SK-500 | 350 | " | 2000 | 4.6 | 3.9 | 100 | — | |
| 31 | " | " | 360 | " | 2000 | 4.3 | 7.5 | 100 | — | |
| 32 | " | " | 370 | " | 2000 | 4.3 | 9.4 | 96 | 4 | |
| 33 | " | " | 380 | " | 2000 | 4.2 | 12.1 | 92.6 | 5.4 | |
| 34 | " | " | 390 | " | 2000 | 4.0 | 12.8 | 81.9 | 4.5 | 13.6 |
| 35 | " | " | 400 | " | 2000 | 3.8 | 13 | 69 | 2.0 | 28.9 |

0 101 921

TABLE 1 (Cont'd)
Amination of olefins

| Run | Olefin | Catalyst | T, °C | bar | GHSV | $NH_3$/ olefin | Conv. (%) | % Selectivity | | |
|---|---|---|---|---|---|---|---|---|---|---|
| | | | | | | | | Monoamine | Diamine | Other |
| 36 | Ethylene | H-Erionite | 360 | 54 | 2000 | 3.4 | 9.3 | 88.5 | 10.7 | 0.8 |
| 37 | " | " | 375 | " | 2000 | 8.0 | 15.5 | 88.4 | 10.5 | 1.1 |
| 38 | " | " | 380 | " | 2000 | 3.5 | 12.3 | 84.7 | 13.8 | 1.5 |
| 39 | " | " | 380 | " | 2000 | 5.2 | 9.9 | 88.5 | 8.8 | 2.7 |
| 40 | " | " | 380 | " | 2000 | 4.1 | 13.5 | 83.3 | 12.3 | 2.6 |
| 41 | " | " | 380 | " | 2000 | 4.7 | 12.9 | 87.8 | 10.0 | 2.2 |
| 42 | " | " | 380 | " | 2000 | 5.5 | 13.0 | 88.4 | 8.7 | 2.9 |
| 43 | " | " | 380 | " | 2000 | 5.6 | 14.4 | 87 | 8.5 | 4.5 |
| 44 | " | " | 390 | " | 2000 | 5.8 | 15.1 | 86.2 | 8.4 | 5.0 |
| 45 | " | " | 390 | " | 2000 | 5.8 | 16.3 | 84.3 | 8.6 | 5.6 |
| 46 | " | " | 400 | " | 2000 | 5.7 | 17.5 | 77.7 | 7.7 | 14.6 |
| 47 | " | " | 400 | " | 2000 | 5.8 | 17.9 | 78.6 | 7.9 | 13.2 |
| 48 | " | " | 320 | " | 2000 | 3.8 | 1.6 | 90.8 | 3.7 | 5.5 |
| 49 | " | " | 320 | " | 2000 | 4.3 | 2.8 | 95.4 | 4.5 | — |

TABLE 1 (Cont'd)

| Run | Olefin | Catalyst | T, °C | bar | GHSV | NH₃/olefin | Conv. (%) | % Selectivity | | |
|-----|--------|----------|-------|-----|------|-----------|-----------|------------|---------|-------|
| | | | | | | | | Monoamine | Diamine | Other |
| 50 | " | " | 340 | " | 2000 | 4.7 | 3.5 | 97.2 | 2.7 | — |
| 51 | " | " | 360 | " | 2000 | 4.5 | 7.4 | 92.4 | 6.7 | 0.8 |
| 52 | " | " | 360 | " | 2000 | 3.0 | 8.7 | 88.6 | 10.5 | 1.0 |
| 53 | " | " | 370 | " | 2000 | 2.7 | 10.2 | 86.2 | 12.5 | 1.2 |
| 54 | " | " | 380 | " | 2000 | 3.2 | 13.4 | 85.4 | 11.7 | 1.7 |
| 55 | " | " | 380 | " | 2000 | 4.0 | 15.2 | 85.7 | 12.5 | 2.3 |
| 56 | " | " | 400 | " | 2000 | 3.9 | 16.5 | 84.2 | 13.1 | 2.5 |
| 57 | Propylene | " | 320 | " | 2000 | 4.1 | 0.14 | 100 | — | |
| 58 | Propylene | " | 340 | " | 2000 | 4.5 | 0.46 | 100 | — | |
| 59 | Propylene | " | 360 | " | 2000 | 5.0 | 1.5 | 90.7 | — | |
| 60 | Propylene | " | 380 | " | 2000 | 4.6 | 2.1 | 89.8 | — | |
| 61 | Propylene | " | 400 | " | 2000 | 4.7 | 2.7 | 75.5 | 17.8 | |
| 62 | Propylene | H-Clinoptilolite | 320 | " | 2000 | 4.0 | 0.13 | 68 | — | |
| 63 | Propylene | " | 340 | " | 2000 | 3.9 | 0.34 | 100 | — | |
| 64 | Propylene | " | 360 | " | 2000 | 4.1 | 1.0 | 88.1 | — | |

TABLE 2

Comparison of ethylene conversion and amine selectivity as a function of temperature

| Temp. | SK-500* | | H-clinoptilolite | | H-errionite | | H-offretite | |
|---|---|---|---|---|---|---|---|---|
| | A | B | A | B | A | B | A | B |
| 340 | | | 5.6 | 100 | 5.26 | 100 | 7.7 | 99.8 |
| 350 | 8.3 | 100 | | | | | | |
| 360 | 5.3 | 100 | 9.7 | 100 | 8.76 | 99.2 | 12.3 | 99.7 |
| 370 | 8.0 | 100 | 11.7 | 100 | 12.52 | 98.7 | 17.1 | 99.6 |
| 380 | 10.3 | 98 | 12.6 | 100 | 11.13 | 97.8 | 17.2 | 98.8 |
| 390 | 10.8 | 86.4 | 14.6 | 100 | | | | |
| 400 | 11.0 | 71 | 15.3 | 99.6 | 17.79 | 94 | | |

*=% $C_2H_4$ conversion was corrected by introducing correction factors of 0.7 and 0.6 for $C_2H_4$ and MEA respectively.
A=% ethylene conversion.
B=selectivity to ethylamines.

The data in Tables 1 and 2 shows that the H-offretite and H-erionite consistently gave better selectivity and better conversion than SK-500 at a given temperature and mole ratio, etc. The data in Table 1 also shows generally better conversion for H-offretite than H-clinoptilolite or H-erionite at a given temperature. (Note Runs 3, 4, 8, 25, 26, 33—35, 38, 44—47, 55 and 56.) Runs 9—19, particularly, runs 12—14 and 57—64 show that H-offretite is much more effective for aminating propylene than H-clinoptilolite and H-erionite. Because of this ability, H-offretite offers an advantage over previous catalyst systems in that it can be used to aminate multiple olefin feedstocks without a catalyst charge. H-clinoptilolite and H-erionite are not effective for propylene.

Table 2 shows high conversion for H-offretite at temperatures from 370—380°C. Although there were instances were high conversions were obtained with other catalysts, H-offretite on the average, gave better results.

From the data in the tables it is clear that the H-offretite is extremely effective for the amination of ethylene, and it is more effective than the standard Linde SK-500 zeolite (a rare earth exchanged Y zeolite), H-erionite and H-clinoptilolite. Conversions were significantly higher than were achieved with the H-clinoptilolite and H-erionite catalysts at temperatures from about 370—400°C. In those cases where conversions were forced to a higher level with SK-500, the corresponding selectivity to the desired amine products was lower than were higher conversions were achieved using clinoptilolite and erionite, note runs 19—30 and Table 2. Another interesting point about H-offretite is that, like H-clinoptilolite and H-erionite, only a small fraction of the reaction product was non-amine, even at high conversions, whereas higher by-product levels were experienced with SK-500.

Statement of industrial application

This invention permits the formation of lower alkyl ($C_{2-4}$) amines by the reaction of relatively inexpensive raw materials. The lower alkyl amines have numerous uses as chemicals for the manufacture of herbicides, as catalysts and other uses.

**Claim**

A method for the production of amines from a reaction mixture comprising a $C_{2-4}$ olefin and ammonia in the vapor phase with a catalyst comprising an acidic alumino silicate under amination conditions which include a temperature in the range of about 300—400°C, a pressure in the range of about 43—78 bar, a mole ratio of ammonia to $C_{2-4}$ olefin of about 1—10:1 and a gas hourly space velocity of about 500—5000, characterized in that as acidic alumino silicate an acidic offretite which is rare earth or hydrogen ion exchanged is used.

**Patentanspruch**

Verfahren zur Herstellung von Aminen aus einem Reaktionsgemisch, enthaltend ein $C_{2-4}$ Olefin und Ammoniak, in der Dampfphase mit einem Katalysator, enthaltend ein saures Aluminiumsilikat, unter

**0 101 921**

Verwendung von Aminierungsbedingungen, die eine Temperatur im Bereich von etwa 300 bis 400°C, einen Druck im Bereich von etwa 43 bis 78 bar, ein Molverhältnis von Ammoniak zu $C_{2-4}$ Olefin von etwa 1 bis 10:1, sowie eine Gas-Transportgeschwindigkeit von etwa 500 bis 5000 umfassen, dadurch gekennzeichnet, daß man als saures Aluminiumsilikat einen sauren Offretit, der mit einem seltenen Erdmetall- oder Wasserstoffion ausgetauscht wurde, verwendet.

**Revendication**

Procédé de fabrication d'amines à partir d'un mélange réactionnel comprenant une oléfine $C_{2-4}$ et de l'ammoniaque en phase vapeur avec un catalyseur comprenant un alumino silicate acide sous des conditions d'amination comprenant une température dans la gamme d'environ 300—400°C, une pression dans la gamme d'environ 43—78 bars, un rapport molaire entre l'ammoniaque et l'oléfine $C_{2-4}$ d'environ 1—10:1 et une vitesse spatiale horaire du gaz d'environ 500—5.000, caractérisé en ce qu'on utilise comme alumino silicate acide une offrétite acide qui a été soumise à échange d'ion au moyen d'une terre rare ou d'hydrogène.